# EUROPEAN PATENT SPECIFICATION

(11) **EP 2 254 579 B1**
(45) Date of publication and mention of the grant of the patent: **10.01.2018**
(21) Application number: 09712762.5
(22) Date of filing: 17.02.2009
(51) Int. Cl.: A61K 31/137, A61K 31/40, A61K 31/445, A61K 31/4453, A61K 31/451, A61K 31/4515, A61K 31/454, A61K 31/46, A61K 31/5415, A61K 31/495, A61P 25/02, A61P 29/02

(54) **USE OF COMPOUNDS BINDING TO THE SIGMA RECEPTOR LIGANDS FOR THE TREATMENT OF NEUROPATHIC PAIN DEVELOPING AS A CONSEQUENCE OF CHEMOTHERAPY**
VERWENDUNG VON VERBINDUNGEN ZUR BINDUNG AN DIE SIGMA-REZEPTOR-LIGANDEN ZUR BEHANDLUNG VON NEUROPATHISCHEN SCHMERZEN INFOLGE VON CHEMOTHERAPIE
UTILISATION DE COMPOSÉS DE LIAISON DE LIGANDS DES RÉCEPTEURS SIGMA POUR LE TRAITEMENT DE LA DOULEUR NEUROPATHIQUE SE DÉVELOPPANT SUITE À LA CHIMIOTHÉRAPIE

(30) Priority: 18.02.2008 EP 08384001; 08.05.2008 EP 08008682
(43) Date of publication of application: 01.12.2010
(73) Proprietor: Laboratorios del Dr. Esteve, S.A., 08041 Barcelona (ES)
(72) Inventor: BAEYENS-CABRERA, José Manuel, 18012 Granada (ES); BUSCHMANN, Helmut, H., 08960 Sant Just Desvern (ES); VELA-HERNANDEZ, José-Miguel, 08028 Barcelona (ES); ZAMANILLO-CASTANEDO, Daniel, 08041 Barcelona (ES); NIETO-LÓPEZ, Francisco Rafael, 18012 Granada (ES)
(74) Representative: Peters, Hajo
(86) International application number: PCT/EP2009/001109
(87) International publication number: WO 2009/103487

(56) References cited:
- WO-A-2006/010587
- WO-A-2007/025613
- POLOMANO R C ET AL: "Pain and neuropathy in cancer survivors: Surgery, radiation, and chemotherapy can cause pain; research could improve its detection and treatment" CANCER NURSING, LIPPINCOTT-RAVEN PUB., HAGERSTOWN, MD, US, vol. 29, no. 2, suppl, 1 March 2006 (2006-03-01), pages 39-47, XP009107315 ISSN: 0162-220X
- "Chemotherapy at home, pain and its treatment" SOINS, OFFICE DE PUBLICITE GENERALE, PARIS, FR, no. 528, 1 September 1989 (1989-09-01), pages 17-20, XP009107313 ISSN: 0038-0814
- EPSTEIN J B ET AL: "Oral topical doxepin rinse: analgesic effect in patients with oral mucosal pain due to cancer or cancer therapy", ORAL ONCOLOGY, ELSEVIER SCIENCE, OXFORD, GB, vol. 37, no. 8, 1 December 2001 (2001-12-01), pages 632-637, XP004307898, ISSN: 1368-8375, DOI: 10.1016/S1368-8375(01)00005-7
- DATABASE BIOSIS [Online] BIOSCIENCES INFORMATION SERVICE, PHILADELPHIA, PA, US; July 2002 (2002-07), HAMMACK JULIE E ET AL: "Phase III evaluation of nortriptyline for alleviation of symptoms of cis-platinum-induced peripheral neuropathy", Database accession no. PREV200200442268 & HAMMACK JULIE E ET AL: "Phase III evaluation of nortriptyline for alleviation of symptoms of cis-platinum-induced peripheral neuropathy", PAIN, vol. 98, no. 1-2, July 2002 (2002-07), pages 195-203, ISSN: 0304-3959

## Description

### Field of the invention

The present invention refers to the use of compounds binding to the sigma receptor for the treatment or prevention of neuropathic pain resulting from chemotherapy.

### Background of the invention

The treatment of pain conditions is of great importance in medicine. There is currently a world-wide need for additional pain therapy. The pressing requirement for a specific treatment of pain conditions is documented in the large number of scientific works that have appeared recently in the field of applied analgesics.

PAIN is defined by the International Association for the Study of Pain (IASP) as "an unpleasant sensory and emotional experience associated with actual or potential tissue damage, or described in terms of such damage" (IASP, Classification of chronic pain, 2nd Edition, IASP Press (2002), 210). Although pain is always subjective, its causes or syndromes can be classified. The most relevant pain subtypes discussed in this invention are neuropathic pain, allodynia, hyperalgesia, and especially, peripheral neuropathy.

WO 2007/025613 describes the use of compounds which bind to sigma receptors to treat pain associated with diabetes.

WO 2006/010587 discloses the use of compounds which bind to sigma receptors to treat mechanical allodynia induced by capsaicin or mechanical stimulus (Von Frey filaments).

On the other hand, cancer and its associated therapies are some of the biggest health concerns in the world. Chemotherapy, in combination with or as an alternative to surgery, is the method of choice in most cases for controlling or helping patients struck by carcinomas.

Chemotherapy is defined as the use of chemical substances to treat disease and, in the sense of this invention, refers primarily to the use of cytotoxic drugs, called chemotherapeutic drugs, to treat cancer. Chemotherapy in cancer treatment consists of a personalized combination of potent chemotherapy drugs, designed to slow rapid cancer tumor growth, shrink tumors, kill cancer cells, and prevent the spread of cancer. The chemotherapeutic drugs prevent cells from replicating in the typical, out-of-control manner in which cancer cells divide.

Peripheral neurotoxicity is a clinically significant complication of cancer chemotherapy. For several of the most effective drugs (e.g. taxanes, vinca alkaloids, cisplatin, bortezomib, thalidomide and lenolidamide), neurotoxicity is dose-limiting and sometimes forces the termination of otherwise successful therapy (Polomano and Bennett, 2001; Park et al., 2008). Since these drugs are the treatment of choice for a multitude of hematologic malignancies and solid tumours, hundred of thousands of patients are affected each year. Sensory abnormalities from antineoplastic-evoked neurotoxicity range from mild paresthesiae or dysesthesiae in many patients, and in some, in chronic painful peripheral neuropathy (Quasthoff and Hartung, 2002). The occurrence and severity of the neuropathy is dependent on single dose intensity, duration of treatment, cumulative dose, prior or concurrent treatment with other neuropathic drugs and co-existing conditions such as diabetes and alcohol abuse (Alberts et al., 1995; Postma et al., 1995; Forsyth et al., 1997; Quasthoff and Hartung, 2002). It is known in the art that neuropathic pain, allodynia, hyperalgesia, and especially, peripheral neuropathy, develop in a considerable number of cases as a result of chemotherapy. These are very specific symptoms arising from the neurotoxicity of the chemotherapeutic drug. The treatment of these symptoms is crucial for preserving the quality of life of the afflicted patients (Mielke et al., 2006; Park et al., 2008; Argyriou et al., 2008). Unfortunately, an effective treatment for chemotherapy-induced peripheral neuropathy has yet to be found (Wolf et al., 2008).
- Epstein et al. (Oral Oncology 37 (2001) 632-637) describe the analgesic effect of a doxapin rinse in oral mucosal pain due to cancer therapy.
- Hammack et al. (Pain 98 (2001) 195-203) reported that nortryptiline was tried but failed to show a signifcant clinical effect on chemotherapy-related neuropathy.

Therefore, the objective of the present invention is to provide a new form of treatment for neuropathic pain, allodynia, hyperalgesia, and especially, peripheral neuropathy, developing as a consequence of chemotherapy.

This invention demonstrates surprisingly that the administration of a compound binding to the sigma receptor is highly effective for the treatment of neuropathic pain, allodynia or hyperalgesia developing after chemotherapy. This benefit of the invention is more evident when the sigma ligand is specifically a sigma receptor antagonist in the form of a (neutral) antagonist, an inverse agonist or a partial antagonist. Even more surprisingly this invention demonstrates that the co-administration of a sigma ligand and a chemotherapeutic drug prevents the pain onset frequently associated to chemotherapy.

The present invention relates to the use of a compound binding to the sigma receptor - with an IC50 value of ≤ 50nM - for the production of a medicament for the treatment of pain developing as a consequence of chemotherapy. The present invention also relates to the use of a compound binding to the sigma receptor for the production of a medicament for the prevention or treatment of pain developing as a consequence of chemotherapy. Preferably, the pain to be treated is neuropathic pain, allodynia or hyperalgesia. More preferably, the pain to be treated is peripheral neuropathic pain, allodynia, causalgia, hyperalgesia, hyperesthesia, hyperpathia, neuralgia, neuritis or neuropathy.

In one preferred embodiment of the invention, the compound binding to the sigma receptor is used for the prevention of the development of pain developing as a consequence of chemotherapy.

In another preferred embodiment of the invention, the compound binding to the sigma receptor is used for the treatment of pain developing as a consequence of chemotherapy.

"Chemotherapy", in the sense of this invention, is defined as the use of a chemotherapeutic drug for the treatment of cancer, tumors or malign neoplasia.

"Developing as a consequence of chemotherapy" according to this invention is defined as: a) developing after or with at the beginning of chemotherapy and b) thus coinciding with or following the use of a chemotherapeutic drug. Therefore, the symptom to be treated is likely to be caused by or is due to the toxicity, citotoxicity or especially, the peripheral neurotoxicity, of the chemotherapeutic drug.

"Chemotherapeutic drugs" in the sense of this invention are compounds used in chemotherapy, especially those that impair mitosis (cell division) by targeting fast-dividing cells effectively. As these drugs cause damage to cells, they are termed cytotoxic. Some drugs cause cells to undergo apoptosis (so-called "cell suicide"). Preferred chemotherapeutic drugs in the sense of this invention are drugs derived from platin, especially the platin-derivatives cisplatin, carboplatin and oxaliplatin; plant alkaloids and terpenes (terpenoids). Further preferred chemotherapeutic drugs in the sense of this invention are bortezomib, thalidomide and its derivatives, especially lenolidamide.

"Plant alkaloids" (and terpenoids) are alkaloids derived from plants that block cell division by preventing microtubule function. Since microtubules are vital for cell division, their inhibition also arrests cell mitosis. The main examples of plant alkaloids are vinca alkaloids and taxanes.

"Vinca alkaloids" bind to specific sites on tubulin, inhibiting the assembly of tubulin into microtubules (M phase of the cell cycle). They are derived from the Madagascar periwinkle, *Catharanthus roseus* (formerly known as *Vinca rosea*). The vinca alkaloids include Vincristine, Vinblastine, Vinorelbine, and Vindesine.

"Taxanes" are derived from the Pacific yew tree, *Taxus brevifolia.* Taxanes enhance the stability of microtubules, preventing the separation of chromosomes during anaphase. Taxanes include Paclitaxel and Docetaxel.

Examples of chemotherapeutic drugs (by their trademarks), including paclitaxel (Taxol®), Iressa, Gefintinib and Xyotax are:
13-cis-Retinoic Acid, 2-CdA, 2-Chlorodeoxyadenosine, 5-fluorouracil 5-FU, 6-Mercaptopurine, 6-MP, 6-TG 6-Thioguanine, Abraxane, Accutane ®, Actinomycin-D, Adriamycin ®, Adrucil ®, Agrylin ®, Ala-Cort ®, Aldesleukin, Alemtuzumab, ALIMTA, Alitretinoin, Alkaban-AQ ®, Alkeran ®, All-transretinoic acid, Alpha interferon, Altretamine, Amethopterin, Amifostine, Aminoglutethimide, Anagrelide, Anandron ®, Anastrozole, Arabinosylcytosine, Ara-C, Aranesp ®, Aredia ®, Arimidex ®, Aromasin ®, Arranon ®, Arsenic trioxide, Asparaginase, ATRA, Avastin ®, Azacitidine,,BCG, BCNU, Bevacizumab, Bexarotene, BEXXAR ®, Bicalutamide, BiCNU, Blenoxane ®, Bleomycin, Bortezomib, Busulfan, Busulfex ®,,C225, Calcium Leucovorin, Campath ®, Camptosar ®, Camptothecin-11, Capecitabine, Carac TM, Carboplatin, Carmustine, Carmustine wafer, Casodex ®, CC-5013, CCNU (o), CDDP (t), CeeNU (t), Cerubidine (t), cetuximab, Chlorambucil, Cisplatin, Citrovorum Factor, Cladribine, Cortisone, Cosmegen (t), CPT-11 (o), Cyclophosphamide, Cytadren (t), Cytarabine, Cytarabine liposomal, Cytosar-U (t), Cytoxan ®, Dacarbazine, Dactinomycin, Darbepoetin alfa, Daunomycin, Daunorubicin, Daunorubicin hydrochloride (t), Daunorubicin liposomal, DaunoXome (t), Decadron, Delta-Cortef (t), Deltasone (t), Denileukin, diftitox, DepoCyt (t), Dexamethasone, Dexamethasone acetate, dexamethasone sodium phosphate, Dexasone (t), Dexrazoxane, DHAD (o), DIC (t), Diodex (t), Docetaxel, Doxil (t), Doxorubicin, Doxorubicin liposomal, Droxia (t), DTIC, DTIC-Dome (t), Duralone (t), Efudex (t), Eligard (t), Ellence (t), Eloxatin (t), Elspar (t), Emcyt (t), Epirubicin, Epoetin alfa, Erbitux, Erlotinib, Erwinia L-asparaginase (t), Estramustine, Ethyol, Etopophos (t), Etoposide, Etoposide phosphate (t), Eulexin (t), Evista (t), Exemestane, Fareston (t), Faslodex (t), Femara ®, Filgrastim, Floxuridine, Fludara (t), Fludarabine, Fluoroplex (t), Fluorouracil, Fluorouracil (cream), Fluoxymesterone, Flutamide, Folinic Acid (o), FUDR (t), Fulvestrant, G-CSF (t), Gefitinib, Gemcitabine, Gemtuzumab ozogamicin, Gemzar (t), GleevecTM, Gliadel wafer (t), GM-CSF (o), Goserelin, granulocyte - colony stimulating factor (t), Granulocyte macrophage colony stimulating factor (o), Halotestin (t), Herceptin (t), Hexadrol (t), Hexalen (t), Hexamethylmelamine (t), HMM (t), Hycamtin (t), Hydrea (t), Hydrocort Acetate (t), Hydrocortisone, Hydrocortisone sodium phosphate, Hydrocortisone sodium succinate, Hydrocortone phosphate (t), Hydroxyurea, Ibritumomab, Ibritumomab Tiuxetan, Idamycin ®, Idarubicin Ifex ®, IFN-alpha, Ifosfamide, IL-11, IL-2, Imatinib mesylate, Imidazole Carboxamide, Interferon alfa, Interferon Alfa-2b (PEG conjugate) (o), Interleukin - 2 (t), Interleukin-11 (o), Intron A® (interferon alfa-2b), Iressa ®, Irinotecan, Isotretinoin, Kidrolase (t), Lanacort (t), L-asparaginase (t), LCR (o), Lenalidomide (Lenolidamide), Letrozole, Leucovorin, Leukeran (t), Leukine (t), Leuprolide, Leurocristine (o), Leustatin (t), Liposomal Ara-C (t), Liquid Pred (t), Lomustine, L-PAM (o), L-Sarcolysin (o), Lupron (t), Lupron Depot (t), Matulane (t), Maxidex (t), Mechlorethamine, Mechlorethamine Hydrochloride, Medralone (t), Medrol ®, Megace (t), Megestrol, Megestrol Acetate (o), Melphalan, Mercaptopurine, Mesna, Mesnex (t), Methotrexate, Methotrexate Sodium (o), Methylprednisolone, Meticorten (t), Mitomycin, Mitomycin-C (o), Mitoxantrone, M-Prednisol (t), MTC (o), MTX (o), Mustargen (t), Mustine, Mutamycin (t), Myleran (t), Mylocel (t), Mylotarg (t), Navelbine (t), Nelarabine, Neosar (t), Neulasta (t), Neumega (t), Neupogen (t), Nexavar ®, Nilandron (t), Nilutamide, Nipent ®, Nitrogen Mustard (o), Novaldex (t), Novantrone (t), Octreotide, Octreotide acetate (o), Oncospar (t), Oncovin (t), Ontak (t), Onxal (t), Oprevelkin, Orapred (t), Orasone (t), Oxaliplatin, Paclitaxel, Paclitaxel Protein-bound, Pamidronate, Panretin (t), Paraplatin (t), Pediapred (t), PEG Interferon, Pegaspargase, Pegfilgrastim, PEG-INTRON (t), PEG-L-asparaginase, PEMETREXED, Pentostatin, Phenylalanine Mustard (o), Platinol (t), Platinol-AQ (t), Prednisolone, Prednisone, Prelone (t), Procarbazine, PROCRIT ®, Proleukin (t), Prolifeprospan 20 with Carmustine implant (t), Purinethol (t), Raloxifene, Revlimid ®, Rheumatrex (t), Rituxan (t), Rituximab, Roferon-A®, (interferon alfa-2a) Rubex (t), Rubidomycin hydrochloride (t), Sandostatin ®, Sandostatin LAR (t), Sargramostim, Solu-Cortef (t), Solu-Medrol (t), Sorafenib, STI-571, Streptozocin, SU11248, Sunitinib, Sutent ®, Tamoxifen, Tarceva ®, Targretin (t), Taxol ®, Taxotere (t), Temodar ®, Temozolomide, Teniposide, TESPA (o), Thalidomide, Thalomid ®, TheraCys (t), Thioguanine, Thioguanine Tabloid (t), Thiophosphoamide (o), Thioplex (t), Thiotepa, TICE ®, Toposar (t), Topotecan, Toremifene, Tositumomab, Trastuzumab, Tretinoin, Trexall (t), Trisenox (t), TSPA (o), VCR (o), Velban (t), Velcade ®, VePesid (t), Vesanoid (t), Viadur (t), Vidaza (t), Vinblastine, Vinblastine Sulfate (o), Vincasar Pfs (t), Vincristine, Vinorelbine, Vinorelbine tartrate (o), VLB (o), VM-26 (o), VP-16 (t), Vumon (t), Xeloda ®, Xyotax, Zanosar (t), Zevalin TM, Zinecard (t), Zoladex ®, Zoledronic acid, and Zometa ®.

Another drugs used in cancer-therapy (mostly as chemotherapeutics) are:
(as trademarks): Aldara, Alimta, Androcur, Arimidex, Borea, Caelyx, Campto, Casodex, Decapeptyl, Eloxatin, Eutirox, Faslodex, Femara, Gemzar, Gonapeptyl, Grisetin, Herceptin, Isovorin, Lysodren, Megefren, Metvix, Navelbine, Novaldex, Novantrone, Paraplatin, Procrin, Prostacur, Suprefact, Tamoxifeno Funk, Taxol, Taxotere, Testex, Elmu/Prolongatum, Tomudex, Utefos, Vepesid, Xeloda, Zoladex;
(as active compounds): Anastrozole, Bicalutamide, Busereline, Capecetabine, Cisplatin, Carboplatin, Desoxorubicin, Docetaxel, Etoposid, Fulvestrant, Gemcitabine, Gosereline, Irinotecan, Letrozole, Leuproreline, Megestrol, Mitotane, Mitoxantrone, Oxaliplatin, Paclitaxel, Pemetrexed, Raltitrexed, Tamoxiphen, Tegafur, Triptoreline, Vincristine, Vinblastine, Vinorelbine, and Vindesine.

Paclitaxel (Taxol®) is one of the most effective and commonly used antineoplastic drugs for the treatment of solid tumours. It has two serious side effects, myelosupression and peripheral neurotoxicity. The granulocyte colony-stimulating factor effectively counteracts the neutropenia in most patients. However, there are no acceptable therapies to prevent or minimize the nerve damage, making neurotoxicity a significant dose-limiting side effect (Rowinsky et al., 1993a, b; Wasserheit et al., 1996; Gordon et al., 1997; Mielke et al., 2006). Paclitaxel-induced neurotoxicity typically presents as a sensory neuropathy, with the most common complaints being numbness, tingling, burning pain and cold allodynia (Rowinsky et al., 1993a; Chaudhry et al., 1994; Forsyth et al., 1997; Dougherty et al., 2004). Sensory symptoms usually start symmetrically in the feet, but sometimes appear simultaneously in both hands and feet (Rowinsky et al., 1993a; Quasthoff and Hartung, 2002; Mielke et al., 2006). A clinically significant number of patients with paclitaxel-induced neuropathy experience neuropathic pain. For example, in a study of 27 patients treated with paclitaxel doses of 135, 175 and 250-300 mg/m², neuropathic symptoms occurred in 50, 79 and 100% of patients, progressing to dose-limiting neurotoxicity in 0, 21 and 71% of patients, respectively (Postma et al., 1995).

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention this term is to be treated as synonymous to "Neurogenic Pain" which is defined by the IASP as "pain initiated or caused by a primary lesion, dysfunction or transitory perturbation in the peripheral or central nervous system". Neuropathic pain according to this invention is restricted to the neuropathic pain resulting from chemotherapy, meaning caused by the use of a chemotherapeutic drug in chemotherapy. The most likely cause of this pain is the chemotherapeutic drug neurotoxicity, and more specifically, its peripheral neurotoxicity.

According to the IASP "allodynia" is defined as "a pain due to a stimulus which does not normally provoke pain" (IASP, Classification of chronic pain, 2nd Edition, IASP Press (2002), 210). According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful" (IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

According to the IASP "neuralgia" is defined as "pain in the distribution of a nerve or nerves" (IASP, Classification of chronic pain, 2nd Edition, IASP Press (2002), 212).

According to the IASP "neuritis" is defined as "inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

"The sigma receptor/s" as used in this application is/are well known and defined using the following citation: "this binding site represents a typical protein different from opioid, NMDA, dopaminergic, and other known neurotransmitter or hormone receptor families" (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequences of the sigma receptors (Sigma 1 (σ1) and Sigma 2 (σ2)) are known in the art (e.g. Prasad, P.D. et al., J. Neurochem. 70 (2), 443-451 (1998)). They show a very high affinity to various analgesics (e.g. pentazocine).
"Compound/s binding to the sigma receptor" or "sigma ligand" as used in this application is/are defined as a compound having an IC₅₀ value of ≤ 50 nM. Additionally, the wording "Compound/s binding to the sigma receptor", as used in the present application is defined as having at least ≥50% displacement using 10 nM radioligand specific for the sigma receptor (e.g. preferably [³H]-(+) pentazocine) whereby the sigma receptor may be any sigma receptor subtype. Preferably, said compounds bind to the sigma-1 receptor subtype.

Compounds binding to the sigma receptor, generally also referred to as sigma ligands, are well known in the art. Many of them are encompassed by the "Compound/s binding to the sigma receptor" definition above. Although there are many known uses for sigma ligands, such as antipsychotic drugs, anxiolytics, antidepressants, stroke treatment, antiepileptic drugs and many other indications, including anti-migraine and general pain, there is no mention in the art of these compounds as useful for the treatment of the symptoms of pain developing as a consequence of chemotherapy.

Table 1 lists sigma ligands known in the art (having an IC₅₀ ≤ 50 nM). Some of these compounds may bind to the sigma-1 and/or to the sigma-2 receptor. These sigma ligands also include their respective salts, bases, and acids.

**Table 1**

| | |
|---|---|
| 4-(4-Fluorobenzoyl)-1-(4-Phenylbutyl)Piperidine Oxalate | |
| | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | |
| | Astemizole |
| | BD 1008 DiHBr |
| BD-1047 | BD-1063 |
| Benproperine Phosphate | |
| | Bromhexine HCl |
| | Bromperidol |
| Cis-(+/-)-N-Methyl-N-[2-(3,4-Dichlorophenyl)Ethyl]-2-(1-Pyrrolidinyl)Cyclohexamine DiHBr | |
| | Flunarizine diHCl |
| GBR 12909 DiHCl | |
| GBR-12935 DiHCl | |
| | HEAT HCl |
| | Ifenprodil Tartrate |
| L-693,403 Maleate | |
| L-741,742 HCl | |
| | Lobeline HCl |
| Iomerizine diHCl | |
| | MR 16728 HCl |
| | NE-100 |
| | PAPP |
| RS 67333 HCl | |
| Safranin O HCl | |
| | Thonzonium Bromide |
| | Trifluperidol HCl |
| | Trimeprazine Hemi-L-Tartrate |

Preferably, the table above includes also reduced haloperidol. Reduced haloperidol is an active metabolite of haloperidol that is produced in humans, shows a high affinity (in the low nanomolar range) for sigma-1 receptors, and produces an irreversible blockade of sigma-1 receptors both in experimental animals and human cells.

In this application "about" means "approximately", and illustratively, the use of the term "about" indicates that dosages slightly outside the cited ranges may also be effective and safe. Compounds that are "administered together with compounds binding to the sigma receptor" or "in combination with compounds binding to the sigma receptor" may be administered as part of the same composition, or may be administered separately, at the same or at separate times, in the same therapeutic regimen.
In connection with this invention "neutral form" refers either to a non-ionic form or to a neutrally net charged form, for example a Zwitter-Ion at its isoelectric point.
The term "salt" according to this invention is to be understood as meaning any form of the active compound according to the invention in which this compound assumes an ionic form or is charged and, if applicable, is also coupled to a counter-ion (a cation or anion). By "salt" is should also be understood complexes of the active compound with other molecules and ions, and in particular complexes which are formed via ionic interactions. Preferred examples of salts include those formed by acetate, mono-trifluoracetate, acetate ester salt, citrate, formate, picrate, hydrobromide, monohydrobromide, monohydrochloride or hydrochloride ions and molecules.

The term "physiologically acceptable salt" in the context of this invention is understood as meaning a salt of at least one of the compounds according to the invention which are physiologically tolerated by humans and/or mammals.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

The term "treatment" or "to treat" in the context of this specification means administration of a compound or formulation according to the invention to prevent, ameliorate or eliminate one or more symptoms associated with neuropathic pain, hyperalgesia and/or allodynia.

Furthermore, the terms "to treat" or "treatment" according to this invention include the treatment of symptoms of neuropathic pain, hyperalgesia and/or allodynia, the prevention or the prophylaxis of the symptoms of neuropathic pain, hyperalgesia and/or allodynia, as well as the prevention or prophylaxis of the causes of the neuropathic pain, hyperalgesia and/or allodynia symptoms.

According to the various embodiments of the invention, the compounds binding to the sigma receptor or the pharmaceutical compositions comprising them, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said compound binding to the sigma-receptor as active ingredient with the above mentioned carriers, followed by placing the mixture into a hard gelatin capsule or soft capsule. Also, said compound binding to the sigma-receptor may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In certain embodiments of the invention a formulation or pharmaceutical composition may contain an active ingredient (a compound binding to the sigma receptor) as well as optionally at least one auxiliary material and/or additive. In other embodiments of the invention the formulation or pharmaceutical composition may contain optionally one or more additional active ingredients.

In certain embodiments of the invention the auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples of these include parenteral formulations such as intravenous subcutaneous or intramuscular application formulations, which could also be applied by other administration routes.

In certain embodiments of the invention the routes of administration of the compound binding to the sigma receptor include intramuscular injection, intraveneous injection, subcutaneous injection, sublingual, bucal, patch through skin, oral ingestion, implantable osmotic pump, collagen implants, aerosols or suppository.

In alternative embodiments of the invention the compounds binding to the sigma receptor may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In some embodiments of the invention the effectiveness of a course of treatment of one, two, three, four, five or more doses or one, two or three days may last for up to about five, ten, fifteen, twenty, twenty five or thirty. In other embodiments of the invention dosing is only performed once every day or once every two, three, four, five, six, seven, eight, nine, ten, eleven, twelve, thirteen, fourteen, sixteen, eighteen, twenty, twenty four, thirty or more days.

According to the present disclosure, the dosage of the compound binding to the sigma receptor depends on a variety of factors, including the nature and severity of the diseases, the sex, age, weight and individual reaction of the subject, the particular compound employed, the route and frequency of administration, etc. Said compounds binding to the sigma receptor or the pharmaceutical compositions comprising them may be administered in single or divided dosage form, e.g. one to four doses per day. Those skilled in the art will readily understand and implement the changes to the treatment methods exemplified herein that are necessary or desirable to reflect varied therapeutic requirements.

In a highly preferred embodiment of the invention the neuropathic pain is peripheral.

According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

In another preferred embodiment of the invention the neuropathic pain is allodynia.

According to the IASP "allodynia" is defined as "a pain due to a stimulus which does not normally provoke pain" (IASP, Classification of chronic pain, 2nd Edition, IASP Press (2002), 210).

In another preferred embodiment of the invention the neuropathic pain is causalgia.

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

In another preferred embodiment of the invention the neuropathic pain is hyperalgesia.

According to the IASP "hyperalgesia" is defined as "an increased response to a stimulus which is normally painful" (IASP, Classification of chronic pain, 2nd Edition, IASP Press (2002), 211).

In another preferred embodiment of the invention the neuropathic pain is hyperesthesia.

According to the IASP "hyperesthesia" is defined as "increased sensitivity to stimulation, excluding the senses" (IASP, Classification of chronic pain, 2nd Edition, IASP Press (2002), 211).

In another preferred embodiment of the invention the neuropathic pain is hyperpathia.

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

In another preferred embodiment of the invention the neuropathic pain is neuralgia.

According to the IASP "neuralgia" is defined as "pain in the distribution of a nerve or nerves" (IASP, Classification of chronic pain, 2nd Edition, IASP Press (2002), 212).

In another preferred embodiment of the invention the neuropathic pain is neuritis.

According to the IASP "neuritis" is defined as "inflammation of a nerve or nerves" (IASP, Classification of chronic pain, 2nd Edition, IASP Press (2002), 212).

In another preferred embodiment of the invention the neuropathic pain is neuropathy/neuritis.

According to the IASP "neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

In another preferred embodiment of the invention the neuropathic pain is orofacial pain.

Another aspect of the present invention relates to the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in the form of a salt, especially a physiologically acceptable salt, or in the form of a solvate, especially a hydrate for the treatment of allodynia developing as a consequence of chemotherapy.

Another aspect of the present invention relates to the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in the form of a salt, especially a physiologically acceptable salt, or in the form of a solvate, especially a hydrate for the production of a medicament for the treatment of hyperalgesia developing as a consequence of chemotherapy.

In a highly preferred embodiment of the invention at least one chemotherapeutic agent used in chemotherapy is selected from a platin-derivative, a vinca alkaloid or a taxane. In another highly preferred embodiment of the invention at least one chemotherapeutic agent used in chemotherapy is selected from a platin-derivative, a vinca alkaloid, a taxane, bortezomib, thalidomide or its derivatives.

In a highly preferred embodiment of the invention at least one chemotherapeutic agent used in chemotherapy is selected from the group consisting of cisplatin, carboplatin and oxaliplatin; vincristine, vinblastine, vinorelbine and vindesine; paclitaxel and docetaxel. In another highly preferred embodiment of the invention at least one chemotherapeutic agent used in chemotherapy is selected from the group consisting of cisplatin, carboplatin and oxaliplatin; vincristine, vinblastine, vinorelbine and vindesine; paclitaxel and docetaxel; bortezomib; thalidomide and lenolidamide.

Preferably, at least one chemotherapeutic agent used in chemotherapy is cisplatin.

Preferably, at least one chemotherapeutic agent used in chemotherapy is carboplatin.

Preferably, at least one chemotherapeutic agent used in chemotherapy is oxaliplatin.

Preferably, at least one chemotherapeutic agent used in chemotherapy is vincristine.

Preferably, at least one chemotherapeutic agent used in chemotherapy is vinblastine.

Preferably, at least one chemotherapeutic agent used in chemotherapy is vinorelbine.

Preferably, at least one chemotherapeutic agent used in chemotherapy is vindesine.

Preferably, at least one chemotherapeutic agent used in chemotherapy is paclitaxel.

Preferably, at least one chemotherapeutic agent used in chemotherapy is docetaxel.

Preferably, at least one chemotherapeutic agent used in chemotherapy is bortezomib.

Preferably, at least one chemotherapeutic agent used in chemotherapy is thalidomide.

Preferably, at least one chemotherapeutic agent used in chemotherapy is lenolidamide.

This invention also includes (non-claimed) methods for the treatment of a patient or a mammal, including human, suffering from neuropathic pain developing as a consequence of chemotherapy that involve using a compound binding to the sigma receptor, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in the form of a salt, especially a physiologically acceptable salt, or in the form of a solvate, especially a hydrate. A preferred (non_claimed) method of treatment according to the present invention comprises the application of compound binding to the sigma receptor selected from Tables 1 or 2 or otherwise metioned herein to a patient or mammal undergoing or about to undergo chemotherapy. It is also preferred if the (non-claimed) method of treatment comprises applying at least one chemotherapeutic agent selected from a platin-derivative, a vinca alkaloid or a taxane; especially if at least one chemotherapeutic agent is selected from the group consisting of cisplatin, carboplatin and oxaliplatin; vincristine, vinblastine, vinoreibine and vindesine; and paclitaxel and docetaxel. It is preferred as well if the (non-claimed) method of treatment comprises applying at least one chemotherapeutic agent selected from a platin-derivative, a vinca alkaloid, a taxane, bortezomib, thalidomide or its derivatives; especially if at least one chemotherapeutic agent is selected from the group consisting of cisplatin, carboplatin and oxaliplatin; vincristine, vinblastine, vinorelbine and vindesine; paclitaxel and docetaxel; bortezomib; thalidomide and lenolidamide.

The (non-claimed) method of treatment according to the present invention includes also the co-administration of a chemotherapeutic drug or drugs jointly or in combination with a compound binding to the sigma receptor during chemotherapy. The co-administration may be perfomed before, during or after chemotherapy. The co-administrarion could also be periodic or uninterrupted.

Another highly preferered embodiment of the invention is the treatment or prevention of pain developing as a consequence of chemotherapy, wherein the compound binding to the sigma receptor is combined with a a least one chemotherapeutic drug (B) forming a fixed-dose active substance combination. Preferably, the chemotherapeutic drug (B) in this active substance combination is selected from a platin-derivative, a vinca alkaloid or a taxane, especially if chemotherapeutic drug (B) is selected from cisplatin, carboplatin and oxaliplatin; vincristine, vinblastine, vinorelbine and vindesine; or paclitaxel and docetaxel. It is also preferable if the chemotherapeutic drug (B) in this active substance combination is selected from a platin-derivative, a vinca alkaloid, a taxane, bortezomib, thalidomide or its derivatives; especially if the chemotherapeutic drug (B) from is selected from cisplatin, carboplatin and oxaliplatin; vincristine, vinblastine, vinorelbine and vindesine; paclitaxel and docetaxel; bortezomib; or thalidomide and lenolidamide.

Another alternative embodiment of the present invention refers to a kit comprising a compound binding to the sigma receptor, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in the form of a salt, especially a physiologically acceptable salt, or in the form of a solvate, especially a hydrate.

The orofacial region, the face and mouth, represent sites of some of the most common pains in the body. Epidemiological studies have revealed the high prevalence of several orofacial pain conditions such as temporomandibular disorders (TMD), burning mouth syndrome, and toothaches, (Dworkin, 2001; Feinman and Newton-John, 2004; LeResche, 2001; Lipton et al., 2001). Many of the difficulties experienced by clinicians with the management of acute and chronic orofacial pain conditions stem from a lack of recognition and understanding of their complex factors and interactions, from uncertainties of the aetiology or pathogenesis of many of the conditions, as well as the lack of information of the comparative effectiveness of the analgesic drugs on orofacial pain.

Thus, a preferred aspect of the invention is the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in the form of a salt, especially a physiologically acceptable salt, or in the form of a solvate, especially a hydrate for the treatment of orofacial pain, preferably, in the form of neuropathic pain, hyperalgesia or allodynia, more preferably, in the form of neuropathic pain, hyperalgesia or allodynia, developing as a consequence of chemotherapy.

The following examples and figures are illustrative of certain embodiments of the invention.

### Examples

### Pharmacological Experiments

Recently, models of paclitaxel-induced painful neuropathy have been developed in mice and rats. These models demonstrated that repeated administration of paclitaxel produced mechanical hyperalgesia and allodynia (Authier et al., 2000; Polomano et al., 2001; Dina et al., 2001 y 2004; Smith et al., 2004; Flatters y Bennett, 2004), cold allodynia (Polomano et al., 2001; Smith et al., 2004; Flatters y Bennett, 2004) and in some studies a thermal (warm) hyperalgesia (Polomano et al., 2001; Dina et al., 2001; Flatters y Bennett, 2004); however, other studies did not find this thermal hyperalgesia (Authier et al., 2000; Smith et al., 2004). Nevertheless, paclitaxel-induced painful neuropathy in rodents represents an interesting model to test the effects of drugs in chemotherapy-induced neuropathic pain.

### FIGURES:

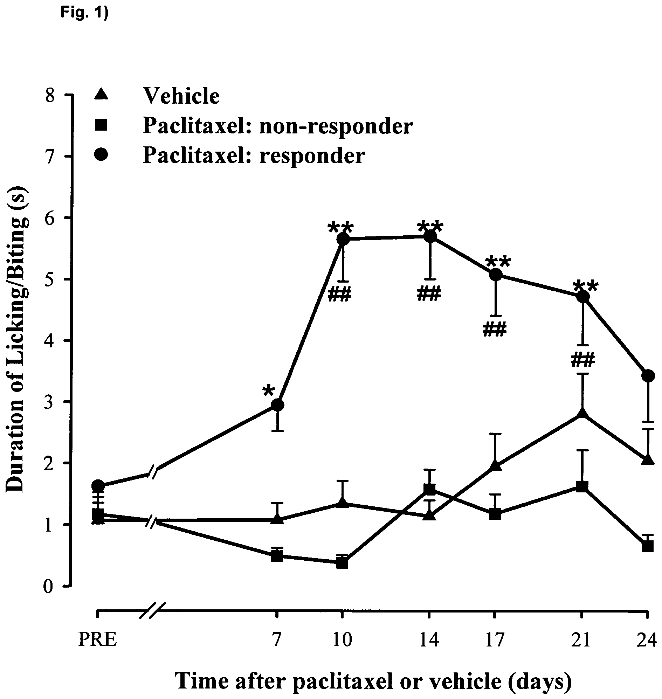

**Fig. 1****:** Time-course of paclitaxel induced cold-allodynia in mice. Animals were treated once daily from days 1 to 5 with paclitaxel (2 mg/kg) or its vehicle via i.p. The duration of hind paw licking/biting in the acetone test was recorded 3 days before (PRE) and at several days after the first injection of paclitaxel or its vehicle. Each animal was tested only in one nociceptive model. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

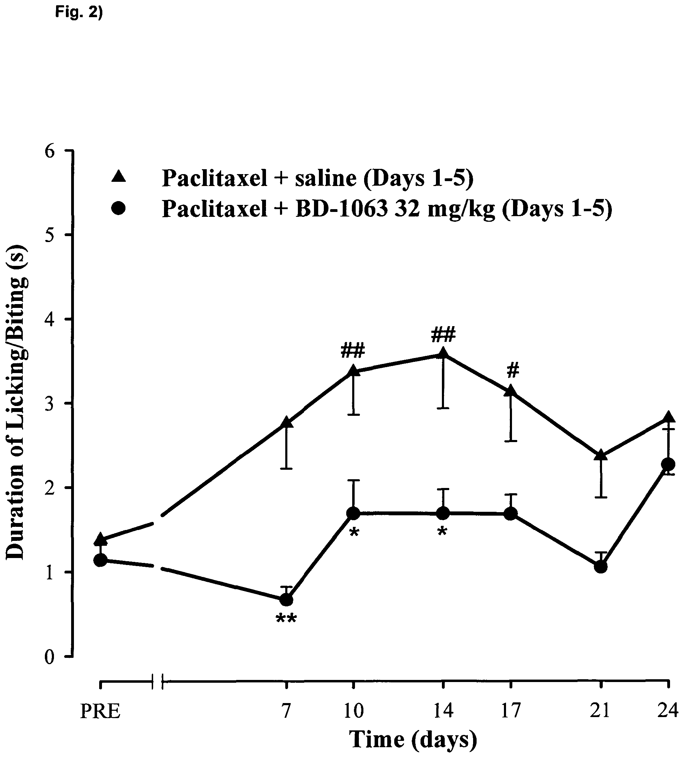

**Fig. 2****:** Time-course of the effect of co-administration of paclitaxel + BD-1063 (32 mg/kg) or paclitaxel + saline on duration of hind paw licking/biting in the acetone test. Mice were treated once daily from days 1 to 5 with an s.c. injection of BD-1063 (32 mg/kg) or saline, 30 min before each i.p. injection of paclitaxel (2 mg/kg). The response evaluated was recorded in each animal 3 days before (PRE) and at several days after the first injection of paclitaxel + BD-1063 or paclitaxel + saline. Each point and vertical line represents the mean ± S.E.M. of the values obtained in at least 16 animals. Statistically significant differences in comparison to paclitaxel + saline: * *p* < 0.05, ** *p* < 0.01; and between the values on pre-treatment day and the days after treatment: # *p* < 0.05, ## *p* < 0.01 (two-way repeated measures ANOVA followed by Newman-Keuls test).

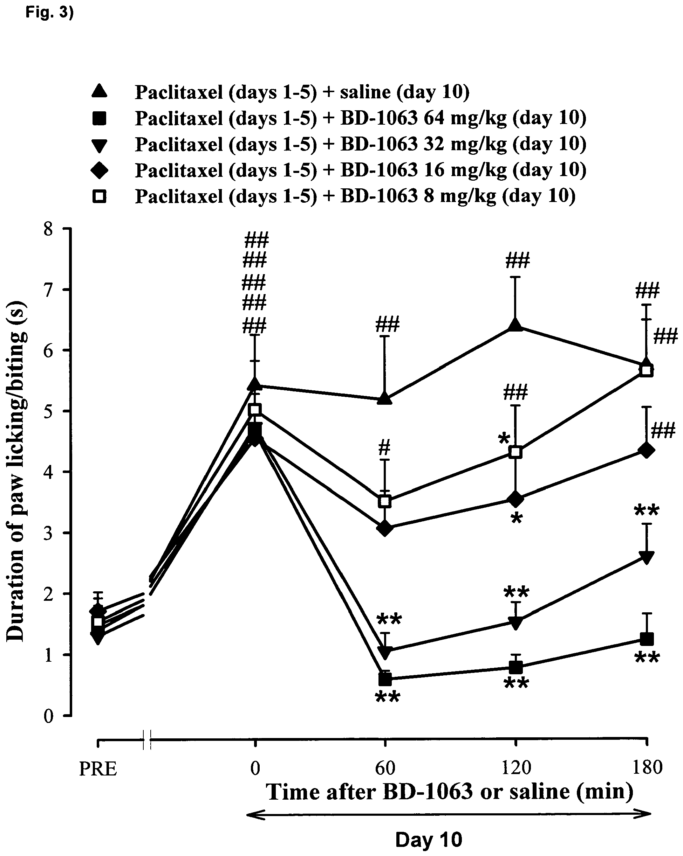

**Fig. 3****:** Effect of a single treatment with several doses of BD-1063 or saline on the duration of hind paw licking/biting (acetone test) on day 10 (a day of maximum effect) in mice pre-treated with paclitaxel. Animals were treated once daily from days 1 to 5 with paclitaxel or its vehicle via i.p. and the day 10 received a single injection of BD-1063 (8, 16, 32 or 64 mg/kg) or saline. The duration of hind paw licking/biting was recorded in each animal 3 days before (PRE) and 10 days after the first injection of paclitaxel or its vehicle. This day, duration of hind paw licking/biting was recorded immediately before (time 0) and at several times (60, 120 and 180 min) after the injection of BD-1063 or saline. Each animal received either saline or one dose of BD-1063. Each point and vertical line represents the mean ± S.E.M. of the values obtained in at least 12 animals. Statistically significant differences between the BD-1063- and saline-treated groups on the same day after treatment: * *p* < 0.05; ** *p* < 0.01; and between the values obtained in the pretreatment day and in day 10 at different times after drug or saline administration: # *p* < 0.05; ## *p* < 0.01 (two-way repeated measures ANOVA followed by Newman-Keuls test).

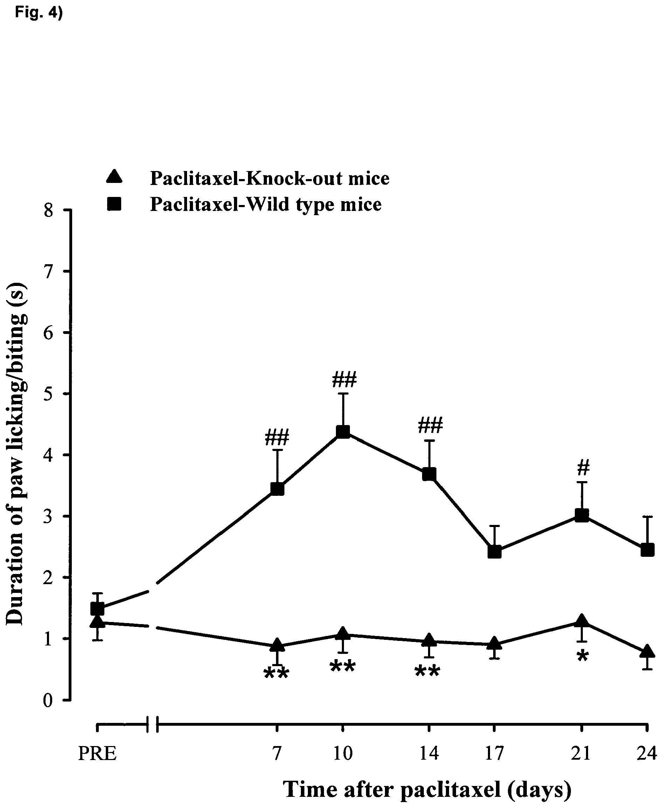

**Fig. 4****:** Time-course of paclitaxel induced cold-allodynia in to groups of mice, one being sigma-1-receptor knock-out mice and the other wild-type mice. Animals were treated once daily from days 1 to 5 with paclitaxel (2 mg/kg) or its vehicle via i.p. The duration of hind paw licking/biting in the acetone test was recorded 3 days before (PRE) and at several days after the first injection of paclitaxel or its vehicle. Each animal was tested only in one nociceptive model. It turned out that only wild-type animals treated with paclitaxel showed increased duration of hind paw licking/biting in the acetone test. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

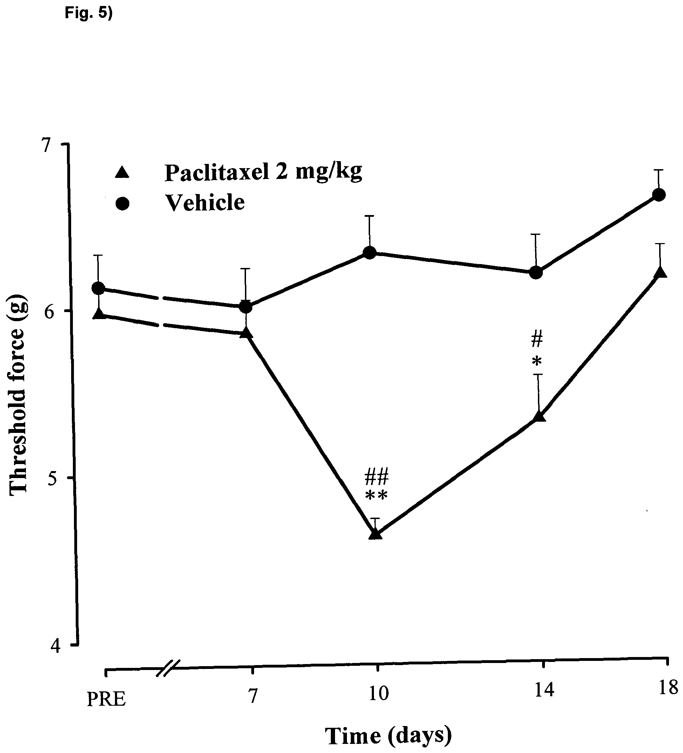

**Fig. 5****:** Time-course of paclitaxel induced mechanical allodynia in mice. Animals were treated once daily from days 1 to 5 with paclitaxel (2 mg/kg) or its vehicle via i.p. The treshold force in the Von-Frey-test was recorded 3 days before (PRE) and at several days after the first injection of paclitaxel or its vehicle. Each animal was tested only in one nociceptive model. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

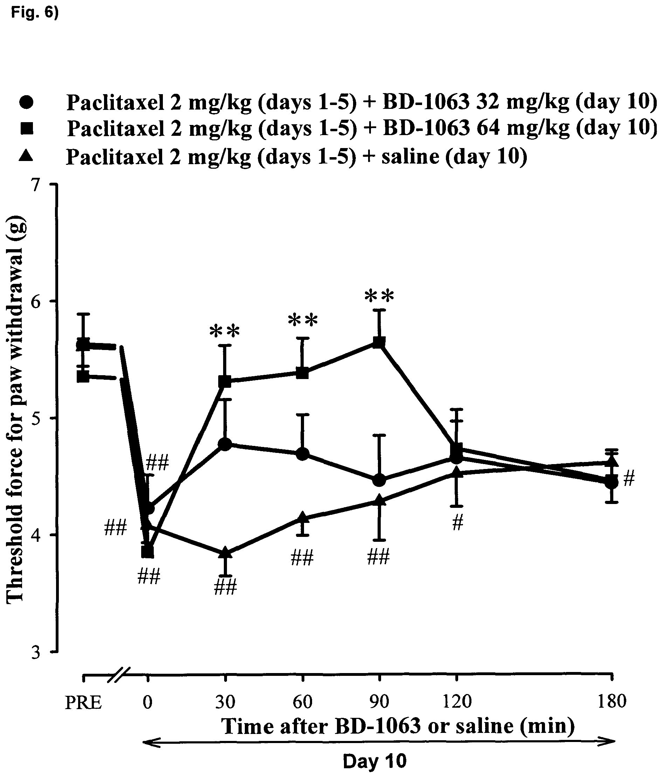

**Fig. 6****:** Effect of a single treatment with several doses of BD-1063 or saline on the threshold force (von-Frey test) to induce hind paw withdrawal on day 10 (a day of maximum effect) in mice pre-treated with paclitaxel. Animals were treated once daily from days 1 to 5 with paclitaxel or its vehicle via i.p. and the day 10 received a single injection of BD-1063 (8, 16, 32 or 64 mg/kg) or saline. The threshold force was recorded in each animal 3 days before (PRE) and 10 days after the first injection of paclitaxel or its vehicle. This day, threshold force was recorded immediately before (time 0) and at several times (60, 120 and 180 min) after the injection of BD-1063 or saline. Each animal received either saline or one dose of BD-1063. Each point and vertical line represents the mean ± S.E.M. of the values obtained in at least 12 animals. Statistically significant differences among the BD-1063- and saline-treated groups at the same time after treatment: ** *p* < 0.01; and between the values obtained in the pretreatment day and in day 10 at different times after drug or saline administration: # *p* < 0.05; ## *p* < 0.01 (two-way repeated measures ANOVA followed by Newman-Keuls test).

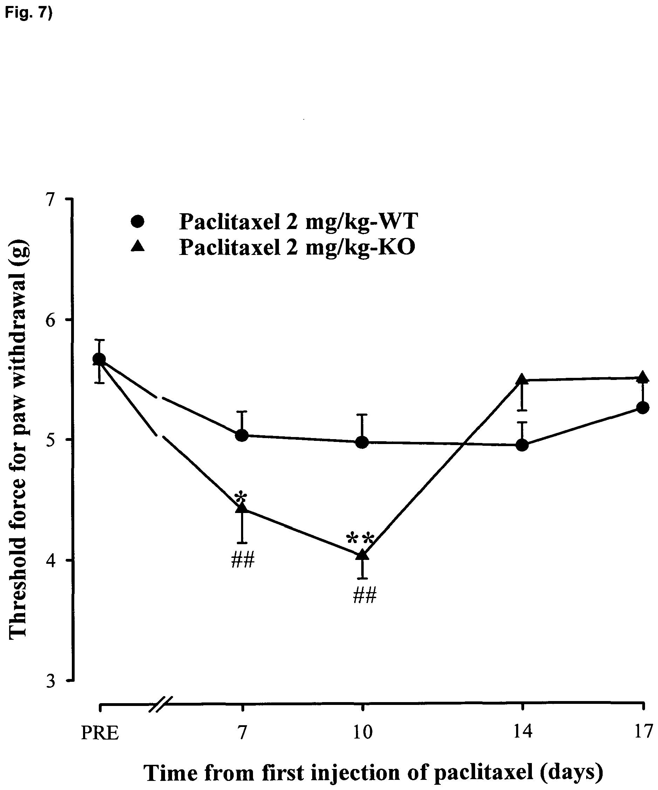

**Fig. 7****:** Time-course of paclitaxel induced mechanical-allodynia in two groups of mice, one being sigma-1-receptor knock-out mice and the other wild-type mice. Animals were treated once daily from days 1 to 5 with paclitaxel (2 mg/kg) or its vehicle via i.p. The threshold force in the von-Frey-test was recorded 3 days before (PRE) and at several days after the first injection of paclitaxel or its vehicle. Each animal was tested only in one nociceptive model. It turned out that only wild-type animals treated with paclitaxel showed decreased threshold strength in the von-Frey test. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

### METHODS:

### In general:

Experiments were performed in CD-1 mice (Charles River, U.S.A.) with at least n = 10/experimental group. Paclitaxel-induced painful peripheral neuropathy was produced by i.p. administration of paclitaxel once daily during 5 days. Control animals received the same volume of solvent (a mixture of ethanol and cremophor EL)

Mechanical allodynia was evaluated with an electronically driven Von Frey filament (Dynamic Plantar Aesthesiometer, Ugo Basile, Varese, Italy) as previously described (Nieto et al., 2008), and cold allodynia was evaluated using the acetone drop method (Polomano et al., 2001; Smith et al., 2004).

A well-known sigma receptor antagonist, BD-1063, was injected s.c. either immediately before each paclitaxel injection to test whether a sigma-antagonist affects the development of the painful peripheral neuropathy or at day 10 (when paclitaxel injections have ended and the neuropathy is fully developed) to test whether BD-1063 interferes with the expression of the different signs of paclitaxel-induced neuropathic pain. In addition, in order to study the influence of the sigma 1 receptor in this process the difference in development of allodynia was determined using wild-type mice and sigma 1-receptor knock-out-mice.

### Specific description:

Mice weighing 25-30 g were used. The animals were housed in colony cages with free access to food and water prior to the experiments. They were maintained in temperature- and light-controlled rooms (22 ± 1 °C, lights on at 08.00 h and off at 20.00 h, air replacement every 20 min). Testing took place during the light phase (from 9.00 h to 15.00 h).

Paclitaxel was dissolved in a solution made up of 50% Cremophor EL and 50% absolute ethanol to obtain a concentration of 6 mg/ml. This paclitaxel solution was conserved at -20 °C during a maximum of 14 days and was diluted in normal saline (NaCl 0.9%), just before administration, to a final concentration of 2 mg/10 ml. The vehicle of paclitaxel was diluted at the time of injection with saline (NaCl 0.9%) in the same proportion as the paclitaxel solution.

Paclitaxel (2 mg/kg) was administered intraperitoneously (i.p.), in a volume of 10 ml/kg, once per day for five consecutive days. Therefore, the cumulative dose was 10 mg/kg per mouse. In the control group the vehicle of paclitaxel was administered following the same schedule. The same schedules of paclitaxel injection also applied when testing groups of sigma-1-knock-out mice against groups of wild-type mice.

BD-1063 was dissolved in normal saline just before administration and applied at doses of 8, 16, 32 or 64 mg/kg subcutaneously.

The effects of BD-1063 on paclitaxel-induced neuropathic pain were examined in two different ways. To evaluate the effect of the sigma receptor antagonist BD-1063 on the development of paclitaxel-induced pain, the animals received an s.c. injection of BD-1063 30 min before each i.p. injection of paclitaxel for five consecutive days. The response of the animals to the different nociceptive stimuli was tested subsequently during 2-4 weeks, depending on the test (see below), without any additional treatment. Each animal was tested only in one nociceptive model. To test the effect of BD-1063 on the *expression* of paclitaxel-induced pain, a single BD-1063 injection was performed on day 10, a day of maximum expression of mechanical allodynia or cold allodynia (see figures for details). Each animal received only one dose of BD-1063 and was tested in only one nociceptive model.

**Procedure for assessment of cold allodynia.** Cold allodynia was tested as previously described by Smith et al., 2004, by gently touching the plantar skin of the hind paws with an acetone bubble formed with a syringe connected to a thin polyethylene tube. The mice were housed and habituated for 30 min in transparent plastic boxes (7 x 7 x 13 cm) with a floor made of wire mesh. After the adaptation period, acetone was applied alternately three times to each paw at intervals of 30 s, and the duration and frequency of licking or biting were recorded. A small mirror was placed behind the chambers to allow clear observation of the paws. The time spent licking or biting the paw was recorded by a stopwatch and represented as the cumulative time of licking/biting in the six measurements. Since licking persisting more than 10 s in the experiments was very unusual, a cut-off time of 10 s was used for each trial.

To elucidate the time-course of paclitaxel-induced cold allodynia in control mice, the animals were tested previously to the paclitaxel administration (pretreatment value, 3 days before first paclitaxel-treatment) and on different days (days 7, 10, 14, 17, 21 and 24) after the first paclitaxel or vehicle injection.

The same procedure was followed to compare sigma-1-knock-out mice to wild type mice, thus elucidating the time-course of paclitaxel-induced cold allodynia in control mice. Accordingly, the animals (two equal groups of knock-out and wild-type mice were tested previously to paclitaxel administration (pretreatment value, 3 days before first paclitaxel-treatment) and on different days (days 7, 10, 14, 17, 21 and 24) after the first paclitaxel or vehicle injection.

The same procedure was followed to test the effect of BD-1063 on the development of cold allodynia, but in this case, BD-1063 or its vehicle was injected s.c. 30 min before each of the 5 paclitaxel i.p. injections. Once more the animals were tested previously to the paclitaxel/BD-1063 administration (pretreatment value, 3 days before first paclitaxel/BD-1063-treatment) and on different days (days 7, 10, 14, 17, 21 and 24) after the first paclitaxel/BD-1063 or vehicle injection. The effect of BD-1063 on the expression of paclitaxel-induced cold allodynia was evaluated on day 10, because the maximum allodynic effect was observed on that day. Therefore, the day 10, after the habituation period to the apparatus, baseline latencies were recorded, 30 min later BD-1063 or saline was injected s.c. and paw withdrawal latencies were assessed again 30, 60, 90, 120 and 180 minutes after the injection. Around 33 % of the control animals treated with paclitaxel did not show cold allodynia; therefore, it was differentiated between 'responders' and 'non-responders' mice in this test. The 'non-responders' mice were easily identified because they spent less than 2 s licking/biting the paw stimulated with acetone on days 7 and 10 after paclitaxel administration. The 'non-responder' animals were not used to test the effect of BD-1063 on the expression of cold allodynia since they do not express enough cold allodynia.

**Procedure for assessment of mechanical allodynia.** To assess mechanical allodynia, paw withdrawal thresholds were measured using a Dynamic Plantar Aesthesiometer (Ugo Basile, Italy). The electronic Von Frey device employs a single nonflexible filament which applies a progressively increasing force (from 0 to 10 g) against the plantar surface of the hind paw over a 20 s period. The nocifensive withdrawal reflex automatically turns off the stimulus and the mechanical threshold value is showed in a screen. The day of the experiment, mice were placed individually in test compartments (9 x 9 x 14 cm) with a wire mesh bottom and allowed to acclimatize to them for 2 h. After habituation, each mouse was tested three times alternately in each hind paw.

To elucidate the time-course of paclitaxel-induced mechanical allodynia in control mice, the animals were tested previously to the paclitaxel administration (pretreatment value; day 3 before paclitaxel-treatment) and on different days (days 7, 10, 14 and 17) after the first paclitaxel or vehicle injection.

The same procedure was followed to compare sigma-1-knock-out mice to wild type mice, thus elucidating the time-course of paclitaxel-induced mechanical allodynia in control mice. Accordingly, the animals (two equal groups of knock-out and wild-type mice were tested previously to the paclitaxel administration (pretreatment value, 3 days before first paclitaxel-treatment) and on different days (days 7, 10, 14, 17, 21 and 24) after the first paclitaxel or vehicle injection.

The same procedure was followed to test the effect of BD-1063, a well-know sigma-receptor antagonist, on the development of paclitaxel-induced mechanical allodynia. In this case, BD-1063 or its vehicle was injected s.c. 30 min before each of the 5 paclitaxel i.p. injections. Once more the animals were tested previously to the paclitaxel/BD-1063 administration (pretreatment value, 3 days before first paclitaxel/BD-1063-treatment) and on different days (days 7, 10, 14, 17, 21 and 24) after the first paclitaxel/BD-1063 or vehicle injection. The effect of BD-1063 on the expression of paclitaxel-induced mechanical allodynia was evaluated on day 10, because the maximum change of the mechanical threshold was observed on that day. Therefore, the day 10, after the habituation period to the apparatus, baseline latencies were recorded, 30 min later BD-1063 or saline was injected s.c. and paw withdrawal latencies were assessed again 30, 60, 90, 120 and 180 minutes after the injection. Most animals (96 %) treated with paclitaxel showed a reduction of the mechanical threshold; those animals that did not show mechanical allodynia were not used to test the effect of BD-1063 on the expression of paclitaxel-induced mechanical allodynia.

### Results

**A) Time-course of paclitaxel-induced cold- and mechanical-allodynia in control mice.** The values obtained on the pre-treatment day in paclitaxel- and vehicle-treated animals were not significantly different in the acetone test and Von Frey test. Administration during 5 days of paclitaxel-vehicle did not significantly modify the response of the animals in any test at any post-treatment day in comparison to the pre-treatment value.
   In the acetone test (Fig. 1), administration of paclitaxel (2 mg/kg, i.p.) once daily during 5 days allowed to distinguish between two groups of animals depending on their response. Most animals (67%) treated with paclitaxel increased significantly (p < 0.01) the time spent licking/biting the paw stimulated (Fig. 1) and the frequency of paw licking/biting at all the post-treatment days, in comparison to the pre-treatment day value. These animals constitute the paclitaxel-responder animals. On the other hand, a 33% of paclitaxel-treated animals did not show cold allodynia, and their response to acetone was indistinguishable from that of animals treated with the paclitaxel-vehicle in both duration (Fig. 1) and frequency of licking/biting. When the values of these two variables among the different groups obtained on the same day of evaluation were compared, statistically significant differences between paclitaxel-responder and the other two groups (paclitaxel-non-responder or paclitaxel-vehicle) were observed for each day of evaluation after treatment (Fig. 1). Paclitaxel-induced cold allodynia was maximal 10-14 days after the first injection of the antineoplastic for both variables recorded (Fig. 1); therefore, the effect of BD-1063 on the expression of cold allodynia was evaluated on day 10.
   Administration of paclitaxel (2 mg/kg, i.p., during 5 days) induced mechanical allodynia in mice, since it significantly reduced the threshold force for paw withdrawal in the Von Frey test on day 10, in comparison with both the pre-treatment day value and the value obtained the same day in the paclitaxel-vehicle treated animals (Fig. 5). Therefore, the effect of BD-1063 on the expression of mechanical allodynia was tested on day 10.
**B) Effect of BD-1063 on the development of paclitaxel-induced cold- and mechanical-allodynia.** The pre-treatment values were similar in the two experimental groups (paclitaxel + saline and paclitaxel + BD-1063) in the acetone test.
   The group of animals in which paclitaxel (i.p.) and saline (s.c.) were co-administered during days 1 to 5 showed a significant increase on duration of paw licking/biting (Fig. 2) in the acetone test, which began on day 7 and became maximum on days 10-14 after the first injection, as in the experiments were only paclitaxel was injected (Fig. 1). On the other hand, in the animals which received an i.p. co-injection of BD-1063 in a dose of 32 mg/kg together with paclitaxel during days 1 to 5, the duration of paw licking/biting (throughout the 24 days after co-administration) were statistically significantly different between the values obtained in both groups (paclitaxel + saline and paclitaxel + BD-1063 (32 mg/kg)) from day 7 onwards, when duration of licking/biting was analysed. Therefore, the co-administration of paclitaxel i.p. and BD-1063 (32 mg/kg) inhibited the development of cold allodynia induced by paclitaxel.
**C) Effect of BD-1063 on the expression of paclitaxel-induced cold allodynia.** The duration and the frequency of paw licking/biting on day 10, before the treatment with BD-1063 or saline, were significantly different from their values on pre-treatment day in all groups of animals treated. As expected, paclitaxel induced a cold allodynia 10 days after its first injection. A single s.c. injection of saline on day 10 did not significantly modify the expression of paclitaxel-induced cold allodynia. The acute treatment with various amounts of BD-1063 (8, 16, 32 or 64 mg/kg) inhibited the expression of paclitaxel-induced cold allodynia. This effect of BD-1063 was dose-dependently, significantly different from that of saline (Fig.3).
**D) Effect of BD-1063 on the expression of paclitaxel-induced mechanical allodynia.** The threshold forces of paw withdrawl on day 10, before the treatment with BD-1063 or saline, were significantly different from their values on pre-treatment day in all groups of animals treated. As expected, paclitaxel induced mechanical allodynia 10 days after its first injection. A single s.c. injection of saline on day 10 did not significantly modify the expression of paclitaxel-induced mechanical allodynia. The acute treatment with various amounts of BD-1063 (32 or 64 mg/kg) inhibited the expression of paclitaxel-induced mechanical allodynia. This effect of BD-1063 was dose-dependently, significantly different from that of saline (Fig.6).
**E) Time-course of paclitaxel-induced cold-allodynia in a comparison between wild-type mice and sigma-1-receptor knockout mice.** In the acetone test (Fig. 4), administration of paclitaxel (2 mg/kg, i.p.) once daily during 5 days resulted in two groups of animals showing different effects. In wild-type animals, paclitaxel induced cold allodynia was maximal 10 days after the first injection of the antineoplastic (Fig. 4). In contrast, in sigma-1-receptor knockout animals, paclitaxel-induced cold allodynia was not significantly expressed (Fig. 4). Therefore, paclitaxel-induced cold allodynia is an effect related to the sigma-1 receptor.
F) **Time-course of paclitaxel-induced mechanical-allodynia in a comparison between wild-type mice and sigma-1-receptor knockout mice.** In the von-Frey test (Fig. 7), administration of paclitaxel (2 mg/kg, i.p.) once daily during 5 days resulted in the two groups of animals showing different effects. In wild-type animals, paclitaxel induced mechanical allodynia was maximal 10 days after the first injection of the antineoplastic (Fig. 7). In sigma-1-receptor knockout animals, paclitaxel-induced mechanical allodynia was not significantly expressed (Fig. 7). Therefore, paclitaxel-induced mechanical allodynia is an effect related to the sigma-1 receptor.

### References:

Alberts DS, Noel JK. Cisplatin-associated neurotoxicity: can it be prevented? Anticancer Drugs. 1995; 6(3):369-83.
Argyriou AA, Iconomou G, Kalofonos HP. Bortezomib-induced peripheral neuropathy in multiple myeloma: a comprehensive review of the literature. Blood 2008; 112(5): 1593-9.
Chaudhry V, Rowinsky EK, Sartorius SE, Donehower RC, Cornblath DR. Peripheral neuropathy from taxol and cisplatin combination chemotherapy: clinical and electrophysiological studies. Ann Neurol. 1994; 35(3):304-11.
Dougherty PM, Cata JP, Cordella JV, Burton A, Weng HR. Taxol-induced sensory disturbance is characterized by preferential impairment of myelinated fiber function in cancer patients. Pain. 2004; 109(1-2): 132-42.
Forsyth PA, Balmaceda C, Peterson K, Seidman AD, Brasher P, DeAngelis LM. Prospective study of paclitaxel-induced peripheral neuropathy with quantitative sensory testing. J Neurooncol. 1997; 35(1):47-53.
Gordon AN, Stringer CA, Matthews CM, Willis DL, Nemunaitis J. Phase I dose escalation of paclitaxel in patients with advanced ovarian cancer receiving cisplatin: rapid development of neurotoxicity is dose-limiting. J Clin Oncol. 1997; 15(5): 1965-73.
Mielke S, Sparreboom A, Mross K. Peripheral neuropathy: a persisting challenge in paclitaxel-based regimes. Eur J Cancer 2006; 42(1):24-30.
Nieto FR, Entrena JM, Cendán CM, Pozo ED, Vela JM, Baeyens JM. Tetrodotoxin inhibits the development and expression of neuropathic pain induced by paclitaxel in mice. Pain 2008; 137(3):520-31.
Park SB, Krishnan AV, Lin CS, Goldstein D, Friedlander M, Kiernan MC. Mechanisms underlying chemotherapy-induced neurotoxicity and the potential for neuroprotective strategies. Curr Med Chem 2008; 15(29):3081-94.
Polomano RC, Bennett GJ. Chemotherapy-evoked painful peripheral neuropathy. Pain Med. 2001; 2(1):8-14.
Postma TJ, Vermorken JB, Liefting AJ, Pinedo HM, Heimans JJ. Paclitaxel-induced neuropathy. Ann Oncol. 1995; 6(5):489-94.
Quasthoff S, Hartung HP. Chemotherapy-induced peripheral neuropathy. J Neurol. 2002; 249(1):9-17.
Rowinsky EK, Eisenhauer EA, Chaudhry V, Arbuck SG, Donehower RC. Clinical toxicities encountered with paclitaxel (Taxol). Semin Oncol. 1993a; 20 (4 Suppl 3):1-15.
Rowinsky EK, Chaudhry V, Forastiere AA, Sartorius SE, Ettinger DS, Grochow LB, Lubejko BG, Cornblath DR, Donehower RC. Phase I and pharmacologic study of paclitaxel and cisplatin with granulocyte colony-stimulating factor: neuromuscular toxicity is dose-limiting. J Clin Oncol. 1993b; 11(10):2010-20.
Smith SB, Crager SE, Mogil JS. Paclitaxel-induced neuropathic hypersensitivity in mice: responses in 10 inbred mouse strains. Life Sci. 2004; 74(21):2593-604.
Wasserheit C, Frazein A, Oratz R, Sorich J, Downey A, Hochster H, Chachoua A, Wernz J, Zeleniuch-Jacquotte A, Blum R, Speyer J. Phase II trial of paclitaxel and cisplatin in women with advanced breast cancer: an active regimen with limiting neurotoxicity. J Clin Oncol. 1996; 14(7):1993-9. Erratum in: J Clin Oncol 1996 Dec; 14(12):3175.
Wolf S, Barton D, Kottschade L, Grothey A, Loprinzi C. Chemotherapy-induced peripheral neuropathy: prevention and treatment strategies. Eur J Cancer 2008; 44(11):1507-15.

## Claims

1. Use of a compound binding to the sigma receptor for the treatment or prevention of pain developing as a consequence of chemotherapy; **characterized in that** said compound binding to the sigma receptor has an IC50 value of ≤ 50 nM.

2. Use, according to claim 1, in which the compound binding to the sigma receptor is used for the prevention of the development of pain developing as a consequence of chemotherapy.

3. Use, according to claim 1, in which the compound binding to the sigma receptor is used for the treatment of pain developing as a consequence of chemotherapy.

4. Use, according to claim 3, in which the pain is neuropathic pain, allodynia or hyperalgesia.

5. Use, according to claim 4, in which the neuropathic pain is selected from peripheral neuropathic pain, allodynia, causalgia, hyperalgesia, hyperesthesia, hyperpathia, neuralgia, neuritis or neuropathy.

6. Use, according to claim 1, **characterized in that** the compound may be in neutral form, in the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable mixing ratio.

7. Use, according to any of claims 1 to 6, **characterized in that** said compound binding to the sigma receptor used is binding to the sigma receptor as an antagonist, a partial antagonist or an inverse agonist.

8. Use, according to any of claims 1 to 9, **characterized in that** said compound binding to the sigma receptor is binding to the sigma-1 receptor subtype.

9. Use, according to claim 1, **characterized in that** said compound binding to the sigma receptor is selected from the group consisting of:
| | |
|---|---|
| 4-(4-Fluorobenzoyl)-1-(4-Phenylbutyl)Piperidine Oxalate | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | Astemizole |
| BD-1047 | BD 1008 DiHBr |
| Benproperine Phosphate | BD-1063 |
| Bromperidol | Bromhexine HCl |
| Cis-(+/-)-N-Methyl-N-[2-(3,4-Dichlorophenyl)Ethyl]-2-(1-Pyrrolidinyl)Cyclohexamine DiHBr | GBR 12909 DiHCl |
| GBR-12935 DiHCl | HEAT HCl |
| L-693,403 Maleate | Ifenprodil Tartrate |
| L-741,742 HCl | Lobeline HCl |
| Iomerizine diHCl | MR 16728 HCl |
| RS 67333 HCl | NE-100 |
| Trifluperidol HCl | PAPP |
| Trimeprazine Hemi-L-Tartrate | |

10. Use, according to any of claims 1 to 9, **characterized in that** at least one chemotherapeutic agent used in the chemotherapy is selected from a platin-derivative, a vinca alkaloid or a taxane, bortezomib or thalidomide and its derivatives.

11. Use, according to claim 10 **characterized in that** at least one chemotherapeutic agent used in the chemotherapy is selected from the group consisting of cisplatin, carboplatin and oxaliplatin; vincristine, vinblastine, vinorelbine and vindesine; paclitaxel and docetaxel, bortezomib, thalidomide and lenolidamide.

12. Use, according to claim 1 for the treatment or prevention of pain developing as a consequence of chemotherapy, wherein the compound binding to the sigma receptor is combined with at least one chemotherapeutic drug (B) forming a fixed-dose active substance combination.

13. Use, according to claim 12, wherein the chemotherapeutic drug (B) is selected from a platin-derivative, a vinca alkaloid or a taxane, bortezomib or thalidomide and its derivatives,
especially the chemotherapeutic drug (B) is selected from cisplatin, carboplatin and oxaliplatin; vincristine, vinblastine, vinorelbine and vindesine; paclitaxel and docetaxel, bortezomib, thalidomide and lenolidamide.

## Patentansprüche

1. Verwendung einer an den Sigma-Rezeptor bindenden Verbindung zur Behandlung oder Prävention von Schmerz, der als Folge einer Chemotherapie auftritt; **dadurch gekennzeichnet, dass** die an den Sigma-Rezeptor bindende Verbindung einen IC50 Wert von ≤ 50 nM aufweist.

2. Verwendung nach Anspruch 1, wobei die an den Sigma-Rezeptor bindende Verbindung zur Prävention der Entwicklung von Schmerz verwendet wird, der als Folge einer Chemotherapie auftritt.

3. Verwendung nach Anspruch 1, wobei die an den Sigma-Rezeptor bindende Verbindung zur Behandlung von Schmerz verwendet wird, der als Folge einer Chemotherapie auftritt.

4. Verwendung nach Anspruch 3, wobei der Schmerz neuropathischer Schmerz, Allodynie oder Hyperalgesie ist.

5. Verwendung nach Anspruch 4, wobei der neuropathische Schmerz ausgewählt ist aus peripherem neuropathischem Schmerz, Allodynie, Kausalgie, Hyperalgesie, Hyperästhesie, Hyperpathie, Neuralgie, Neuritis oder Neuropathie.

6. Verwendung nach Anspruch 1, **dadurch gekennzeichnet, dass** die Verbindung in neutraler Form, in Form einer Base oder Säure, in Form eines Salzes, vorzugsweise eines physiologisch verträglichen Salzes, in Form eines Solvats oder eines Polymorphs und/oder in Form seines Racemats, reiner Stereoisomere, insbesondere Enantiomere oder Diastereomere oder in Form von Gemischen von Stereoisomeren, insbesondere Enantiomeren oder Diastereomeren vorliegen kann, in einem beliebigen geeigneten Mischungsverhältnis.

7. Verwendung nach einem der Ansprüche 1 bis 6, **dadurch gekennzeichnet, dass** die an den verwendeten Sigma-Rezeptor bindende Verbindung als Antagonist, partieller Antagonist oder inverser Agonist an den Sigma-Rezeptor bindet.

8. Verwendung nach einem der Ansprüche 1 bis 9, **dadurch gekennzeichnet, dass** die an den Sigma-Rezeptor bindende Verbindung an den Sigma-1-Rezeptorsubtyp bindet.

9. Verwendung nach Anspruch 1, **dadurch gekennzeichnet, dass** die an den Sigma-Rezeptor bindende Verbindung ausgewählt ist aus der Gruppe bestehend aus:
| | |
|---|---|
| 4-(4-Fluorbenzoyl)-1-(4-phenylbutyl)piperidinoxalat | 4'-Chlor-3-alpha-(diphenylmethoxy)tropan HCl |
| 4-Furan-2-Ylmethyl-piperazin-1-carboxylsäure 2-{4-[3-(2-trifluormethyl-phenolthiazin-10-Yl)-propyl]-piperazin-1-Yl}-ethylester | Astemizol |
| BD-1047 | BD 1008 DiHBr |
| Benproperinphosphat | BD-1063 |
| Bromperidol | Bromhexin HCl |
| Cis-(+/-)-N-methyl-N-[2-(3,4-dichlorphenyl)ethyl]-2-(1-pyrrolidinyl)cyclohexamin DiHBr | GBR 12909 DiHCl |
| GBR-12935 DiHCl | HEAT HCl. |
| L-693, 403 Maleat | Ifenprodiltartrat |
| L-741,742 HCl | Lobelin HCl. |
| Iomerizin diHCl | MR 16728 HCl |
| RS 67333 HCl | NE-100 |
| Trifluperidol HCl | PAPP |
| Trimeprazin Hemi-L-tartrat | |

10. Verwendung nach einem der Ansprüche 1 bis 9, **dadurch gekennzeichnet, dass** mindestens ein bei der Chemotherapie verwendetes chemotherapeutisches Mittel ausgewählt ist aus einem Platinderivat, einem Vincaalkaloid oder einem Taxan, Bortezomib oder Thalidomid und dessen Derivaten.

11. Verwendung nach Anspruch 10, **dadurch gekennzeichnet, dass** mindestens ein bei der Chemotherapie verwendetes chemotherapeutisches Mittel ausgewählt ist aus der Gruppe bestehend aus Cisplatin, Carboplatin und Oxaliplatin; Vincristin, Vinblastin, Vinorelbin und Vindesin; Paclitaxel und Docetaxel, Bortezomib, Thalidomid und Lenolidamid.

12. Verwendung nach Anspruch 1 zur Behandlung oder Prävention von Schmerz, der als Folge einer Chemotherapie entsteht, wobei die an den Sigma-Rezeptor bindende Verbindung mit mindestens einem chemotherapeutischen Arzneimittel (B) kombiniert wird, wodurch eine Wirkstofffixkombination gebildet wird.

13. Verwendung nach Anspruch 12, wobei das chemotherapeutische Arzneimittel (B) ausgewählt ist aus einem Platinderivat, einem Vincaalkaloid oder einem Taxan, Bortezomib oder Thalidomid und dessen Derivaten,
insbesondere das chemotherapeutische Arzneimittel (B) ausgewählt ist aus Cisplatin, Carboplatin und Oxaliplatin; Vincristin, Vinblastin, Vinorelbin und Vindesin; Paclitaxel und Docetaxel, Bortezomib, Thalidomid und Lenolidamid.

## Revendications

1. Utilisation d'un composé se liant au récepteur sigma pour le traitement ou la prévention de la douleur consécutive à une chimiothérapie ; **caractérisée en ce que** ledit composé se liant au récepteur sigma a une valeur de CI50 ≤ 50 nM.

2. Utilisation, selon la revendication 1, dans laquelle le composé se liant au récepteur sigma est utilisé pour la prévention du développement de la douleur consécutive à une chimiothérapie.

3. Utilisation, selon la revendication 1, dans laquelle le composé se liant au récepteur sigma est utilisé pour le traitement de la douleur consécutive à une chimiothérapie.

4. Utilisation, selon la revendication 3, dans laquelle la douleur est une douleur neuropathique, l'allodynie ou l'hyperalgésie.

5. Utilisation, selon la revendication 4, dans laquelle la douleur neuropathique est sélectionnée parmi la douleur neuropathique périphérique, l'allodynie, la causalgie, l'hyperalgésie, l'hyperesthésie, l'hyperpathie, la névralgie, la névrite ou une neuropathie.

6. Utilisation, selon la revendication 1, **caractérisée en ce que** le composé peut être sous une forme neutre, sous la forme d'une base ou d'un acide, sous la forme d'un sel, de préférence un sel physiologiquement acceptable, sous la forme d'un solvate ou d'un polymorphe et/ou sous la forme de son racémate, de ses stéréoisomères purs, notamment des énantiomères ou des diastéréomères ou sous la forme de mélanges de stéréoisomères, notamment d'énantiomères ou de diastéréomères, à tout rapport de mélange approprié.

7. Utilisation, selon l'une quelconque des revendications 1 à 6, **caractérisée en ce que** ledit composé se liant au récepteur sigma utilisé se lie au récepteur sigma comme un antagoniste, un antagoniste partiel ou un agoniste inverse.

8. Utilisation, selon l'une quelconque des revendications 1. à 9, **caractérisée en ce que** ledit composé se liant au récepteur sigma se lie au sous-type de récepteur sigma-1.

9. Utilisation, selon la revendication 1, **caractérisée en ce que** ledit composé se liant au récepteur sigma est sélectionné dans le groupe constitué de :
| | |
|---|---|
| Oxalate de 4-(4-fluorobenzoyl)-1-(4-phénylbutyl)pipéridine | 4'-chloro-3-alpha-(diphénylméthoxy)tropane HCl |
| 2-{4-[3-(2-trifluorométhyl-phénothiazin-10-yl)-propyl]-pipérazin-1-yl}éthyl ester de l'acide 4-furan-2-ylméthyl-pipérazine-1-carboxylique | Astémizole |
| BD-1047 | BD-1008 DiHBr |
| Phosphate de benpropérine | BD-1063 |
| Brompéridol | Bromhexine HCl |
| Cis-(+/-)-N-méthyl-N-[2-(3,4-dichlorophényl)éthyl]-2-(1-pyrrolidinyl)cyclohexamine DiHBr | GBR 12909 DiHCl |
| GBR-12935 DiHCl | HEAT HCl |
| L-693,403 Maléate | Tartrate d'ifenprodil |
| L-741,742 HCl | Lobeline HCl |
| Iomérizine diHCl | MR 16728 HCl |
| RS 67333 HCl | NE-100 |
| Triflupéridol HCl | PAPP |
| Hémi-L-tartrate de triméprazine | |

10. Utilisation, selon l'une quelconque des revendications 1 à 9, **caractérisée en ce qu'**au moins un agent chimiothérapeutique utilisé dans la chimiothérapie est sélectionné parmi un dérivé du platine, un vinca alcaloïde ou un taxane, le bortézomib ou le thalidomide et ses dérivés.

11. Utilisation, selon la revendication 10, **caractérisée en ce qu'**au moins un agent chimiothérapeutique utilisé dans la chimiothérapie est sélectionné dans le groupe constitué du cisplatine, du carboplatine et de l'oxaliplatine ; de la vincristine, de la vinblastine, de la vinorelbine et de la vindésine ; du paclitaxel et du docétaxel, du bortézomib, du thalidomide et du lénolidamide.

12. Utilisation, selon la revendication 1, pour le traitement ou la prévention de la douleur consécutive à une chimiothérapie, dans laquelle le composé se liant au récepteur sigma est combiné avec au moins un médicament chimiothérapeutique (B) formant une combinaison de substances actives à doses fixes.

13. Utilisation, selon la revendication 12, dans laquelle le médicament chimiothérapeutique (B) est sélectionné parmi un dérivé du platine, un vinca alcaloïde ou un taxane, le bortézomib ou le thalidomide et ses dérivés,
notamment le médicament chimiothérapeutique (B) est sélectionné parmi le cisplatine, le carboplatine et l'oxaliplatine ; la vincristine, la vinblastine, la vinorelbine et la vindésine ; le paclitaxel et le docétaxel, le bortézomib, le thalidomide, et le lénolidamide.
